# EUROPÄISCHE PATENTSCHRIFT

(11) **EP 2 128 113 B1**
(45) Veröffentlichungstag und Bekanntmachung des Hinweises auf die Patenterteilung: **01.05.2013**
(21) Anmeldenummer: 09006305.8
(22) Anmeldetag: 09.05.2009
(51) Int. Cl.: C04B 41/00, C04B 41/51, C04B 41/88, C07C 319/12, C07C 323/52, C03C 17/00, C03C 17/06

(54) **Verwendung von Goldverbindungen auf Basis cycloaliphatischer Mercaptoester in keramischen Edelmetallfarben**
Use of gold compounds on the basis of cycloaliphatic mercapto esters in ceramic noble metal colours
Utilisation de composés d'or à base de mercaptoester cycloaliphatique dans des couleurs de métal précieux céramiques

(30) Priorität: 30.05.2008 DE 102008026071
(43) Veröffentlichungstag der Anmeldung: 02.12.2009
(73) Patentinhaber: Heraeus Precious Metals GmbH & Co. KG, 63450 Hanau (DE)
(72) Erfinder: Hesse, Peer, 64295 Darmstadt (DE); Wenzel, Patrick, 63808 Haibach (DE); Wissel, Sabine, 63796 Kahl/Main (DE); Eife, Peter, 63477 Maintal (DE)
(74) Vertreter: Kühn, Hans-Christian

(56) Entgegenhaltungen:
- EP-A- 0 491 143
- EP-A- 0 514 073
- EP-A- 0 668 264
- WO-A-01/40392
- US-A- 4 652 670

## Beschreibung

Die Erfindung betrifft die Verwendung von Goldverbindungen auf Basis cycloaliphatischer Mercaptoester in keramischen Edelmetallfarben.

Gemäß Absatz [0004] der DE 60008298T2 sind Goldmercaptide und Goldsulforesinate für Pastengolde geeignet. Für flüssige Druckfarben bringe aber die Applikation zusammen mit Acrylaten Vorteile (Absatz [0005]). In Absatz [0007] und Anspruch 1 wird dann vorgeschlagen, Verbindungen der allgemeinen Formel Au-SCHR-CO2R' (mit R und R' jeweils Alkyl, Aryl oder H), zusammen mit Acrylaten in flüssigen Keramik-Druckfarben einzusetzen.

Gemäß US 5328769/DE4040446A1 werden von Mercapto-TCD-Alkoholestern abgeleitete Goldverbindungen für keramische Anwendungen beansprucht:

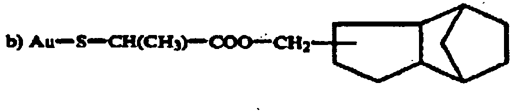

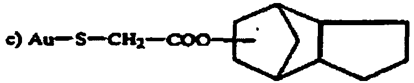

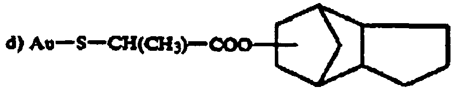

Deren Vorteil bestehe in kürzeren Trocknungszeiten.

Thiole bilden mit Gold sehr stabile Verbindungen. Im Gegensatz zu den aus geschwefelten Naturprodukten hergestellten Goldsulforesinaten oder hydrierten geschwefelten Naturprodukten hergestellten Goldverbindungen, wie z.B. Goldpinanylmercaptid, lassen sich mit den Thiolen eindeutig definierte Verbindungen relativ einfach synthetisieren. Jedoch sind die meisten aromatischen und kurzkettigen Goldmercaptoverbindungen in organischen Lösemitteln sehr schwer löslich. Mit langkettigen aliphatischen Thiolen, die wie bei tert-Dodecylmercaptan aber meistens technisch nur als Isomere verfügbar sind, und mit aliphatischen Mercaptoestern lassen sich zwar lösliche Goldverbindungen herstellen, die aber nur klebrige Massen ergeben. Somit ist die Auswahl der verfügbaren organischen Mercaptoverbindungen zur Herstellung von keramisch nutzbaren Goldverbindungen relativ beschränkt.

Durch Veresterung von Mercaptosäuren und monocyclischen Alkoholen lassen sich Moleküle herstellen, die zum einen feste, leicht isolierbare Goldverbindungen ergeben und zum anderen eine gute Löslichkeit in vielen organischen Lösemitteln zeigen sowie gleichzeitig in keramischen Formulierungen einen glänzenden Goldfilm ergeben. Insbesondere in thermoplastischen Systemen zeigen diese eine sehr gelben und glänzenden Goldfilm und in Pasten verbesserte Trocknungszeiten.

Die vorliegende Erfindung betrifft die Verwendung von Goldverbindungen monocyklischer Mercapto-Alkylester und alkylsubsituierter monoyklischer Mercapto-Alkylester, erhältlich durch
**A** Umsetzung von Thioglykolsäure, Thiomilchsäure und 3-Mercaptopropionsäure mit monozyklischen Alkoholen und
**B** Umsetzung des Reaktionsprodukts mit Gold(I) chloriddialkylsulfidkomplexen in wässriger Lösung und Abtrennung der in Wasser unlöslichen Goldmercaptoesterverbindung,

in nichtflüssigen keramischen Edelmetallfarben.

Nach Schritt **B** erfolgt gegebenenfalls

**C** eine Einarbeitung des Reaktionsprodukts in die Edelmetallfarbe.

Monozyklische Alkohole sind: Cycopentyl-, Cycohexyl-, Cycloheptyl-, Cyclooctyl- und Cylododecylalkohol.

Bevorzugt wird Schritt

**B** durch Umsetzung des Reaktionsprodukts aus Schritt **A** mit Au (I)-Cl · RSR (z.B. R = C₂H₄-OH, Thiodigylkol) durchgeführt.

Besonders bevorzugt ist eine Goldverbindung der Formel

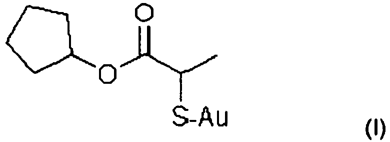

### Herstellung:

Ausgehend von den entsprechenden Alkoholen und den leicht verfügbaren Mercaptosäuren Thioglykolsäure, Thiomilchsäure und 3-Mercaptopropionsäure können Ester synthetisiert werden. In einem nachfolgenden Schritt wird durch Umsetzung mit Au(I)Chlorid die entsprechende Goldverbindung hergestellt und isoliert. Das Au(I)Chlorid ist dabei zweckmäßig mit einer Dithioverbindung stabilisiert

Keramische Edelmetallfarben sind an sich bekannt. Im weiteren Sinne können dazu auch Edelmetallpräparate und Lüsterpräparate gezählt werden. Derartige Zubereitungen sind z.B. in "Dekorprodukte für die Keramik, Aspekte der Produktverantwortung", copyright © 1998: AFN-NECC, CERAMICOLOR, EPSOM, VdMi beschrieben.

Edelmetallpräparate zur Dekoration von Glas, Keramik, Porzellan, Bone-China, Fliesen oder anderen silikatischen Substraten bestehen in der Regel aus Lösungen organischer Gold-, Palladium- und Platinverbindungen (die in zumeist organischen Trägermaterialien gelöst sind), Kunst- oder Naturharzen sowie Flussmitteln, die die Haftfestigkeit auf dem jeweiligen Trägermaterial sicherstellen. Als Flussmittel werden üblicherweise bestimmte organische Metallverbindungen, z. B. Alkoholate, Carboxylate, Resinate oder Sulforesinate von Elementen der dritten bis fünften Hauptgruppe und dritten bis achten Nebengruppe des Periodensystems, besonders der Elemente Rhodium, Silber, Chrom, Wismut, Vanadium, Silicium usw. verwendet. Beim Einbrennen zersetzen sich die organischen Verbindungen zu den jeweiligen Oxiden oder Metallen, wodurch die Haftfestigkeit und optischen Eigenschaften des Metallfilms auf dem Substrat bewirkt werden.

Glanzedelmetallpräparate bestehen im Wesentlichen aus einer oder mehreren in einem flüssigen Trägermedium löslichen Edelmetallverbindungen und Flussmittelverbindungen. Bei den wichtigsten Edelmetallverbindungen handelt es sich um solche von Gold, weshalb derartige Präparate auch häufig als Glanzgoldpräparate bezeichnet werden.

Glanzedelmetallpräparate, wie Glanzgold, Glanzplatin und Glanzpalladium, bestehen aus Lösungen von organischen Gold-, Platin- und Palladiumverbindungen, meist der Sulforesinate, in organischen Verarbeitungshilfsmitteln, die beim Brand restlos verbrennen oder verdampfen, und enthalten üblicherweise noch andere organische Metallverbindungen, beispielsweise die Resinate bzw. Sulforesinate von Rhodium, Wismut und Silber. Die Präparate werden auf den zu dekorierenden Gegenstand aufgetragen und in der Regel bei Temperaturen zwischen 500 und 850 °C eingebrannt. Die auf diese Weise erzeugte Edelmetall-Dekoration kommt hochglänzend aus dem Ofen und braucht daher nachträglich nicht poliert zu werden.

Poliergolde sind Suspensionen feiner Gold-Pulver und/oder schwerlöslicher Goldverbindungen in organischen Verarbeitungshilfsmitteln oder in Glanzgold-Lösungen und können zusätzlich noch lösliche Flussmittel, silikatische Gläser und schwerlösliche Verbindungen enthalten. Polierweißgold-, Poliergold- und Polierplatinpasten enthalten Edelmetall oder Edelmetallverbindungen in fester, feinverteilter und gelöster Form, Haftvermittler sowie Harzlösungen als Filmbildner. Mit Polierpräparaten hergestellte Dekore brennen mit einer stumpfen, bräunlichen Oberfläche aus. Erst durch Polieren mit einer Glasfaserbürste, Sand oder ähnlichen Hilfsmitteln entsteht der für Poliergold- und Polierplatindekore typische seidenmatte Glanz.

Neben diesem optischen Effekt führt die beim Polieren erzielte Verdichtung der Edelmetallpartikel an der Oberfläche des Dekors auch zu einer deutlichen Verbesserung der Abriebfestigkeit. Mit Polierpräparaten hergestellte Dekore sind in der Regel abriebfester als Glanzgolddekore. In Abhängigkeit vom Edelmetallgehalt und der Auftragsstärke bildet sich nach dem Ausbrand ein Goldfilm von etwa 0,3 bis 1,0 µm.

Die Edelmetall-Lüsterpräparate ähneln in ihrer Zusammensetzung den Glanzedelmetallpräparaten, sind jedoch edelmetallärmer und reicher an löslichen organischen Unedelmetallverbindungen als jene. Durch Einbrennen können daraus elektrisch nichtleitende Schichten verschiedener Färbungen erhalten werden. Lüsterpräparate basieren auf in organischen Lösungsmitteln gelösten Metallverbindungen. Nach dem Ausbrand bilden sie eine sehr dünne Schicht (ca. 0,1 µm). Typische Charakteristiken von Lüsterfarben sind ihre Brillanz und der metallischirisierende Glanz, wenn das Präparat auf glatten Substraten ausgebrannt wurde. Auf mattem Untergrund verliert der Lüster seinen irisierenden Effekt und erscheint farbig matt. Lüster eignen sich zur Dekoration von Glas, Porzellan, Bone China, Steingut und Fliesen.

Thermolüster basieren auf in organischen Lösungsmitteln gelösten Metallverbindungen. Nach dem Ausbrand bilden diese blei-, cadmium- und uranfreien Präparate eine sehr dünne Schicht (< 0,1 µm) von hohem Glanz mit metallisch-irisierendem Farbcharakter. Da Thermolüster auf das noch glühende Glas bei vergleichsweise hohen Temperaturen aufgebracht werden, sind sie weitaus abriebfester als bei 500 - 580°C eingebrannte Standardlüsterfarben. Aufgrund des starken Verschmelzens der Lüster mit dem Glas spricht man auch von Diffusion.

Bei den Trägermedien handelt es sich meistens um eine Kombination aus mindestens einem Lösungsmittel und einem Bindemittel. Das flüssige Trägermedium kann rein organisch, organisch-wässrig oder im Wesentlichen rein wässrig sein. Bei den organischen Medien handelt es sich oft um solche auf der Basis von Kohlenwasserstoffen, Alkoholen und schwefelhaltigen Verbindungen, wie geschwefelten Terpenkohlenwasserstoffen und Terpenalkoholen sowie geschwefelten natürlichen Harzen.

Die Dekorherstellung umfasst zumeist einen Brand bei Temperaturen im Bereich von etwa 500 bis 1400 °C.

Die folgenden Beispiele erläutern die Erfindung näher. Teile und Prozentangaben beziehen sich auf das Gewicht, sofern nicht anders angegeben

### Herstellungsbeispiel

Die bevorzugte Verbindung der Formel I wird wie folgt hergestellt:

### Stufe A Ester

19,5 (0,22 mol) Cyclopentanol und 27,6 (0,26 mol) 2-Mercaptopropionsäure werden in 100 ml Toluol gelöst. Als Katalysator werden 0,45 g p-Toluolsulfonsäure zugesetzt. Unter Wärmezufuhr wird die Mischung zum Sieden gebracht. Nach etwa 2 h liegen im Wasserabscheider etwa 4 ml Wasser vor. Die abgekühlte Mischung wird mehrmals mit Wasser bis etwa pH 7 gewaschen. Das Toluol wird abdestilliert. Man erhält etwa 34,5 g Rohester.

### Stufe B Goldmercaptoverbindung

Zur Herstellung der Goldmercaptoverbindung wird im ersten Schritt aus Au(III)-chlorid ein Au(I)-Komplex gebildet. Dazu werden 262,5 g Goldchloridlösung (37,5% Feingold) in eine gekühlte Lösung (<5°C) von g (1 mol) Thiodiglykol zugetropft. Nach Entfärbung der Lösung wird langsam unter weiterer Kühlung der Ester 95,8g (0,55 mol) zugegeben. Es bildet sich ein weißer, gummiartiger Niederschlag, der sich leicht abtrennen lässt. Nach Trocknung kann in Toluol umkristallisiert werden. Man erhält Gold-cyclopentyl-2-mercaptopropionat (170 g Ausbeute 92%, Au-Gehalt Ist 53,18% Soll 53,21 %).

### Anwendungsbeispiel 1

Ein Goldpräparat bestehend aus den Komponenten

| | |
|---|---|
| Gold-cyclopentyl-2-mercaptopropionat | 18,8 Gew. % |
| Silbersulforesinatlösung 42 % | 2,60 Gew. % |
| Rhodiumresinatlösung 5 % | 1,0 Gew. % |
| Iridiumlösung 7 % | 1,0 Gew. % |
| Chromlösung 3 % | 0,6 Gew. % |
| Siliciumresinatlösung 13% | 5,0 Gew. % |
| Polyaminoamidoharz (umgesetzt mit Ethylhexansäure)² | 20,0 Gew. % |
| Pineöl | 39,0 Gew. % |
| Schwefel | 1,0 Gew. % |
| Alkylphenolharz | 8,0 Gew. % |
| Kolophonium | 3,0 Gew. % |

| | |
|---|---|
| (² vgl. DE 103 59 448.5) | |

wird auf ein Abziehbilderpapier aufgebracht, mit Transferlacken auf Basis von Polybutylmethylmethacrylaten/Aromatengemischen überlackiert, auf Porzellan übertragen und bei 820°C gebrannt. Es ergibt sich ein gelber, hochglänzender Goldfilm.

### Anwendungsbeispiel 2

Beispiel eines thermoplastischen Glanzedelmetallpräparates:
Gold-cyclopentyl-2-mercaptopropionat 20,00 Gewichts-%
Siliciumresinat, gelöst in Pineoel (10 % Si) 1,00 Gewichts-%
Rhodiumresinat, gelöst in Pineoel (8 % Rh) 0,40 Gewichts-%
Kupferresinat, gelöst in Pineoel (5 % Cu) 0,20 Gewichts-%
Chromresinat, gelöst in Pineoel (10 % Cr) 1,00 Gewichts-%
Vanadiumresinat, gelöst in Pineoel (2 % V) 2,00 Gewichts-%
Bismutresinat, gelöst in Pineoel (10 % Bi) 1,00 Gewichts-%
Stearylalkohol 49,90 Gewichts-%
Harnstoff-Formaldehydharz, gelöst in Testbenzin (60 %) 7,00 Gewichts-%
geschwefeltes Phenolharz (Schwefelgehalt 20 %) 15,00 Gewichts-%
Silbercarboxylat (39 % Ag) 2,50 Gewichts-%

Die feste, leicht erstarrende Paste wird mit einem beheizten Stahl- oder metallisierten Polyestergewebe auf Trinkgefäße aufgetragen und bei 600 DEG C eingebrannt. Es bilden sich gelbgoldene, hochglänzende Goldfilme mit sehr guter Haftung und hervorragender Spülmaschinenbeständigkeit. Der Goldfilm verändert weder seinen Glanz, Farbton noch seine Spülmaschinenbeständigkeit durch die Angrenzung bzw. geringe Überlappung auf der Farbe.

## Patentansprüche

1. Verwendung von Goldverbindungen monocyclischer Mercapto-Alkylester und alkylsubsituierter monocyclischer Mercaptoester, erhältlich durch
**A** Umsetzung von Thioglykolsäure, Thiomilchsäure und 3-Mercaptopropionsäure mit monozyklischen Alkoholen aus der Gruppe bestehend aus Cyclopentyl-, Cyclohexyl- Cycloheptyl-, Cyclooctyl-und Cylododecylalkohol und deren alkylsubstituierten Abkömmlingen
und
**B** Umsetzung des Reaktionsprodukts mit Gold(I) chloriddialkylsulfidkomplexen in wässriger Lösung und Abtrennung der in Wasser unlöslichen Goldmercaptoesterverbindung,
in nichtflüssigen keramischen Edelmetallfarben.

2. Verwendung nach Anspruch 1, wobei die Goldverbindung der Formel 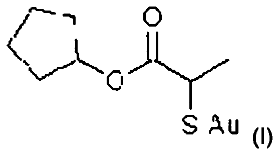 entspricht.

3. Verwendung nach einem der vorstehenden Ansprüche in thermoplastischen oder auf Naturharz basierenden keramischen Edelmetallfarben.

4. Verwendung nach einem der vorstehenden Ansprüche wobei in Schritt **B** mit Au (I)-Cl · RSR umgesetzt wird, wobei R für C₂H₄-OH steht.

5. Verwendung nach einem der vorstehenden Ansprüche, bei der nach Schritt **B C** eine Einarbeitung des Reaktionsprodukts in die Edelmetallfarbe erfolgt.

## Claims

1. Use of gold compounds of monocyclic mercaptoalkyl esters and alkyl-substituted monocyclic mercaptoesters, obtainable through
**A** reacting thioglycolic acid, thiolactic acid, and 3-mercaptopropionic acid with monocyclic alcohols from the group consisting of cyclopentyl, cyclohexyl, cycloheptyl, cyclooctyl, and cyclodecyl alcohol and the alkyl-substituted derivatives thereof
and
**B** reacting the reaction product with gold(I) chloride dialkylsulphide complexes in aqueous solution and separating the water-insoluble gold mercaptoester compound,
in non-liquid ceramic precious metal paints.

2. Use according to claim 1, whereby the gold compound corresponds to formula

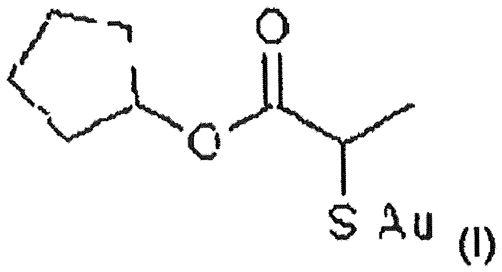

3. Use according to any one of the preceding claims in thermoplastic or natural resin-based ceramic precious metal paints.

4. Use according to any one of the preceding claims, whereby the reaction in **B** involves Au(I)-Cl·RSR, whereby R represents C₂H₄-OH.

5. Use according to any one of the preceding claims, in which after step **B** follows
**C** working the reaction product into the precious metal paint.

## Revendications

1. Utilisation de composés d'or d'esters mercapto-alkyliques monocycliques et de mercaptoesters monocycliques alkyl-substitués, pouvant être obtenus par
**A** conversion d'acide thioglycolique, d'acide thiolactique et d'acide 3-mercaptopropionique avec des alcools monocycliques provenant du groupe constitué de l'alcool cyclopentylique, cyclohexylique, cycloheptylique, cyclooctylique et cyclododécylique et leurs dérivés alkyl-substitués
et
**B** conversion du produit réactionnel avec des complexes de sulfure dialkylique de chlorure d'or (I) en solution aqueuse et séparation du composé de mercaptoester d'or insoluble dans l'eau,
dans des peintures de métaux précieux céramiques non fluides.

2. Utilisation selon la revendication 1, dans laquelle le composé d'or correspond à la formule

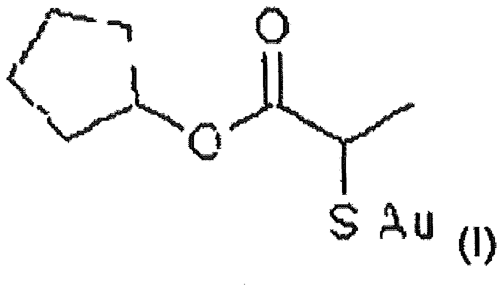

3. Utilisation selon l'une quelconque des revendications précédentes dans des peintures de métaux précieux céramiques thermoplastiques ou à base de résine naturelle.

4. Utilisation selon l'une quelconque des revendications précédentes, dans laquelle la conversion à l'étape **B** a lieu avec Au (I)-CI RSR, dans laquelle R représente C₂H₄-OH.

5. Utilisation selon l'une quelconque des revendications précédentes, dans laquelle **C** une incorporation du produit réactionnel dans la peinture de métaux précieux a lieu après l'étape **B.**
